# EUROPÄISCHE PATENTSCHRIFT

(11) **EP 1 962 923 B1**
(45) Veröffentlichungstag und Bekanntmachung des Hinweises auf die Patenterteilung: **01.05.2013**
(21) Anmeldenummer: 06846962.6
(22) Anmeldetag: 22.12.2006
(51) Int. Cl.: A61M 1/36, B01J 20/22, A61K 35/14, A61K 35/16

(54) **VERWENDUNG EINER MATRIX ZUR ENTFERNUNG VON C-REAKTIVEM PROTEIN AUS BIOLOGISCHEN FLÜSSIGKEITEN**
USE OF A MATRIX FOR REMOVING C-REACTIVE PROTEIN FROM BIOLOGICAL LIQUIDS
UTILISATION D'UNE MATRICE POUR ELIMINER LA PROTEINE C REACTIVE DE LIQUIDES BIOLOGIQUES

(30) Priorität: 22.12.2005 DE 102005061715
(43) Veröffentlichungstag der Anmeldung: 03.09.2008
(73) Patentinhaber: Pentracor GmbH, 16761 Hennigsdorf (DE)
(72) Erfinder: VOGT, Birgit, 13353 Berlin (DE)
(74) Vertreter: Arth, Hans-Lothar
(86) Internationale Anmeldenummer: PCT/DE2006/002335
(87) Internationale Veröffentlichungsnummer: WO 2007/076844

(56) Entgegenhaltungen:
- WO-A-90/12632
- WO-A-03/019135
- WO-A-2004/076486
- GB-A- 2 075 362
- US-A- 4 775 483
- SCHWALBE R A ET AL: "Pentraxin family of proteins interact specifically with phosphorylcholine and/or phosphorylethanolamine" BIOCHEMISTRY, Bd. 31, Nr. 20, 1992, Seiten 4907-4915, XP002432191
- SWANSON S ET AL: "Characteristics of the binding of human C-reactive protein (CRP) to laminin" JOURNAL OF CELLULAR BIOCHEMISTRY, LISS, NEW YORK, NY, US, Bd. 40, Nr. 1, Mai 1989 (1989-05), Seiten 121-132, XP008020876 ISSN: 0730-2312

## Beschreibung

Die vorliegende Erfindung befasst sich mit einer Methode zur Verringerung des Risikos ausgelöst durch erhöhte Mengen von C-reaktivem Protein (CRP), durch die Durchführung einer extrakorporalen Perfusion von Blutplasma von Patienten mit Risiko für kardiovaskuläre Erkrankungen durch ein Gerät, wie z.B. eine Säule, welche absorbierendes Matrixmaterial enthält, beinhaltend Lipide, Peptide, Polypeptide, Phosphocholin (PC) oder PC-Derivate, um C-reaktives Protein zu entfernen. Ferner betrifft die vorliegende Erfindung die Verwendung von Verbindungen zur Herstellung eines pharmazeutischen Mittels, welche die Eigenschaft haben, CRP zumindest temporär zu binden, zur Entfernung von CRP aus biologischen Flüssigkeiten eines Patienten zur Prophylaxe und/oder Behandlung von Immunfehlfunktionen, Autoimmunerkrankungen, kardiovaskulären Erkrankungen, Herzinfarkt, Schlaganfall, Diabetes, Rheuma und Niereninsuffizienz.

### Hintergrund der Erfindung

Kardiovaskuläre Erkrankungen sind eine der hauptsächlichen Todesursachen in den USA und ein Hauptgrund für Morbidität, medizinische Kosten und ökonomischen Verlust für Millionen von Leuten. Zwei der häufigsten und schädlichsten Erscheinungen bei kardiovaskulärer Erkrankung ist das Auftreten von Arteriosklerose und Thrombose.

In den letzten Jahren wurden große Fortschritte in der Behandlung von kardiovaskulären Krankheiten erzielt. Dieser Fortschritt wurde nicht nur durch den Fortschritt der therapeutischen Erkenntnisse der Krankheitsmechanismen ermöglicht, sondern auch durch die frühzeitige Erkennung von Risiko-Patienten für diese Krankheit. Tatsächlich sind die Identifizierung von Risiken für Patienten und die frühzeitige Behandlung wichtige Merkmale der modernen medizinischen Praxis. In den letzten 20 Jahren wurde eine Vielzahl von Faktoren und klinischen Parametern identifiziert, die entweder mit dem gegenwärtigem Zustand oder mit der zukünftigen Wahrscheinlichkeit, eine kardiovaskuläre Krankheit zu entwickeln, korrelieren. Solche Risikofaktoren können meßbare biochemische oder physiologische Parameter beinhalten, wie z. B. Serum-Cholesterin-, HDL-, LDL-, Fibrinogen-Spiegel, usw. oder Verhaltensmuster wie z. B. Übergewicht, Rauchen etc. Der Risikofaktor mit der engsten Verbindung zu der vorliegenden Erfindung ist der Spiegel von C-reaktivem Protein (CRP). CRP wird durch IL-6 induziert.

Die eigentliche Beziehung zwischen einem meßbaren Parameter oder Risikofaktor und dem Krankheitszustand ist nicht immer klar. Mit anderen Worten, es ist nicht immer klar, ob der Risikofaktor an sich ursächlich oder beitragend zur Krankheit ist oder ob er statt dessen eine untergeordnete Erscheinung ist, die auf die Krankheit hinweist. Deshalb könnte eine therapeutische Modalität, die einen Risikofaktor beeinflußt, direkt den pathologischen Krankheitsmechanismus oder den zukünftigen Verlauf beeinflussen, oder könnte sich indirekt günstig auf einen beitragenden Prozess auswirken, der mit der Krankheit zusammenhängt.

Zusätzlich sind viele Risikofaktoren, die mit kardiovaskulärer Erkrankung verbunden sind, in anderen pathologischen Zuständen entweder in einer ursächlichen oder anzeigenden Rolle involviert. Deshalb könnte die Reduktion oder Blockade eines bestimmten Risikofaktors für kardiovaskuläre Erkrankung andere günstige Wirkungen in anderen Krankheiten haben, die mit diesem Risikofaktor verbunden sind.

Von besonderem Interesse für die Methoden der vorliegenden Erfindung ist die Reduktion der kardiovaskulären Risikofaktoren, die mit unnormal hohen CRP-Spiegeln verbunden sind.

CRP wird in der Leber als Antwort auf IL-6 Produktion produziert. IL-6 wird als Teil einer entzündlichen Reaktion im Körper produziert. Deshalb sind CRP- ebenso wie 11-6-Spiegel Marker einer systemischen Entzündungsaktivität. Chronische Entzündung wird als eine der zugrundeliegenden und unterstützenden pathologischen Erscheinungen in kardiovaskulären Erkrankungen vermutet.

In der Menopause, mit dem Verlust des Östrogens, steigt bei Frauen die Prävalenz für kardiovaskuläre Krankheiten. Ebenso nehmen die Risikofaktoren für kardiovaskuläre Krankheiten zu, besonders Lipid- (Cholesterol und Triglyceride), Homocystein- und C-reaktives-Protein (CRP)-Spiegel. Heutzutage ist die Hormon Ersatz Therapie (HRT) die häufigste Methode, um kardiovaskuläre Erkrankungen in Frauen nach der Menopause zu verhindern. Allerdings sind viele Frauen mit dieser Therapie wegen der unangenehmen Nebenwirkungen wie Aufgedunsenheit, Wiederkehr der Mensis, Brustempfindlichkeit, Angst vor Gebärmutter- und Brustkrebs, etc. nicht einverstanden. Außerdem senkt HRT zwar die Cholesterin- und Homocystein-Spiegel erhöht aber die CRP- und IL-6-Spiegel.

Ein Gegenstand der Erfindung ist, ein therapeutisches Agenz zur Verfügung zu stellen, welches diese Risikofaktoren extrakorporal z.B. durch Apherese senkt.

Yeh (Clin Cardiol. 2005 28:408-412) zeigt, daß CRP verwendet werden kann, um das kardiovaskuläre Erkrankungsrisiko voraus zu sagen, daß CRP außerdem ein Indikator für Entzündung ist, und daß Entzündungen alle Stadien der Atherosclerose befördern. Zoccali et al., (Semin Nephrol. 2005 25:358-362) zeigt, daß CRP praediktiv für das kardiovaskuläre Mortalitätsrisiko bei Patienten mit Nierenerkrankung im Endstadium ist. Nurmohamed et al., (Neth J Med. 2005 63:376-381) zeigt, daß CRP prediktiv für kardiovaskuläres Mortalitätsrisiko bei Haemodialysepatienten ist. Trotzdem wird CRP nur als ein Indikatormolekül angesehen.

Sola et al., (J Card Fail. 2005 11:607-612) zeigt, daß Statintherapien dazu genutzt werden können, die Menge an CRP zu senken, und damit die durch kardiovaskuläre Erkrankung hervorgerufene Mortalität und Morbidität reduziert wird. Diese Therapieform genügt aber nicht, um die hohen CRP-Mengen (bis zu 1000fach über normal), welche nach einem Herzinfarkt entstehen, oder die hohen CRP-Mengen in Dialyspatienten signifikant zu reduzieren.

WO 2004/045596 offenbart eine Methode zur Verringerung der Plasma-CRP-Menge, um systemische Entzündungen durch die Verabreichung eines therapeutischen Agens zu reduzieren. Es offenbart aber nicht die extrakorporale Behandlung von biologischen Flüssigkeiten für die Entfernung von CRP aus genannten biologischen Flüssigkeiten.

JP 2002035117 offenbart die Verwendung einer Säule für die Adsorbtion von CRP, um inflammatorische Erkrankungen zu behandeln. Es zeigt auch, daß die Säule inflammatorische Leukozyten entfernt. Die Entfernung von Leukozyten wird in der vorliegenden Erfindung nicht gewünscht, da dies den Patienten zusätzlichen Schaden zufügen würde.

WO 2004/076486 offenbart eine Methode zur Inhibition immunologischer, inflammatorischer und/oder pathophysiologischer Antworten durch Verabreichung von CRP-bindenden Molekülen an Patienten mit erhöhten CRP-Mengen. Aber es offenbart nicht die extrakorporale Behandlung von biologischen Flüssigkeiten zur Entfernung von CRP aus genannten biologischen Flüssigkeiten.

WO 90/12632 offenbart eine Methode zur extrakorporale Behandlung von biologischen Flüssigkeiten zur Entfernung von CRP aus diesen biologischen Flüssigkeiten zur Behandlung von Krebs. Die Matrix kann dabei z.B. aus Silicon, Sepharose, Acrylbeads oder Agarose bestehen und das CRP an Phosphocholin und dessen Derivate gebunden werden.

Aufgabe der vorliegenden Erfindung ist es Mittel und Verfahren zur Prophylaxe und/oder Behandlung von Immunfehlfunktionen, kardiovaskulären Erkrankungen, Herzinfarkt, Schlaganfall, Diabetes, Rheuma und Niereninsuffizienz bereitzustellen.

Diese Aufgabe wird durch die Lehre der unabhängigen Ansprüche gelöst. Weitere vorteilhafte Ausgestaltungen ergeben sich aus der Beschreibung, den Beispielen und den anhängigen Patentansprüchen.

Überraschend wurde die Aufgabe der vorliegenden Erfindung durch die zumindest teilweise Entfernung des C-reaktiven Proteins aus biologischen Flüssigkeiten, insbesondere aus humanem Blut gelöst.

Die vorliegende Erfindung bezieht sich auf die Verwendung von Verbindungen, welche die Eigenschaft haben, humanes CRP zumindest temporär zu binden, zur Herstellung einer pharmazeutischen Matrix zur extrakorporalen Behandlung von biologischen Flüssigkeiten, wie humanes Blut und Blutplasma, Peritoneal-Flüssigkeiten und lymphatischen Flüssigkeiten, welche die Entfernung von C-reaktivem Protein aus besagten biologischen Flüssigkeiten zur Prophylaxe und/oder Behandlung von Herzinfarkt und Schlaganfall umfaßt.

Ein weiterer Aspekt der vorliegenden Erfindung ist auf die Verwendung einer Matrix enthaltend Verbindungen gerichtet, welche die Eigenschaft haben, CRP zumindest temporär zu binden, zur Herstellung eines pharmazeutischen Mittels zur Entfernung von CRP aus biologischen Flüssigkeiten eines Patienten zur Prophylaxe und/oder Behandlung von Herzinfarkt, und . Bei dem pharmazeutischen Mittel handelt es sich vorzugsweise um ein Medizinprodukt in Form einer Adsorbermatrix wie sie für Dialysegeräte bekannt ist. Das Mittel kann daher in Form von Kügelchen, Hochkügelchen, Partikel, porösen Partikel, weitgehend sphärischen Partikeln, weitgehend sphärischen Hochpartikeln, Fasern oder Hohlfasern auftreten. Diese Mittel werden vorzugsweise in entsprechenden Apparaten wie z.B. Säulen oder Dialyseapparaten zwecks Entfernung von CRP aus biologischen Flüssigkeiten eines Patienten eingesetzt.

Mit anderen Worten gesagt, betrifft die Erfindung die Verwendung von Verbindungen, welche die Eigenschaft haben, CRP zumindest temporär zu binden, zur Herstellung einer pharmazeutischen Matrix zur Entfernung von CRP aus biologischen Flüssigkeiten eines Patienten zur Prophylaxe und/oder Behandlung von Herzinfarkt, und Schlaganfall, .

Die kardiovaskulären Erkrankungen werden ausgewählt aus der Gruppe bestehend aus oder umfassend Herzinfarkt, Schlaganfall, Diabetes, Rheuma, Niereninsuffizienz, bluthochdruckbedingte Niereninsuffizienz, Endothel-Verletzungen, Endothel-Zerstörung, Arteriosklerose, Thrombose, Atherosklerose, Stenose, Restenose, atherosklerotische oder thrombotische Erkrankungen, Blutflussinsuffizienz, ischämische Ereignisse, Lungenembolie, stabile und instable Angina pectoris, koronararterielle Erkrankungen, akuter Herztod sowie pathologische Folgen von arteriosklerotischen oder thrombotischen Krankheiten. Insbesondere wichtige und bevorzugte kardiovaskuläre Erkrankungen sind Herzinfarkt, Schlaganfall, Diabetes, Rheuma.

Ferner umfassen die kardiovaskulären Erkrankungen insbesondere akute Endothelschäden und akute Endothelzerstörungen sowie Langzeit-Endothelschäden (chronische Endothelzerstörungen sowie chronische Langzeit-Endothelschäden) und Langzeit-Endothelzerstörungen.

Bei den akuten Endothelschäden und den akuten Endothelzerstörungen handelt es sich um akute Endothelschäden und akute Endothelzerstörungen, vorzugsweise nach Schlaganfall, bei Blutflussinsuffizienz, nach Herzinfarkt, nach akutem Herztod, nach plötzlichem Herztod, bei Brandwunden, bei Operationswunden oder anderen Verletzungen mit teilweise großen Wundarealen, bei diabetischem Schock, bei akutem Leberversagen, bei Pankreatitis, bei neurodegenerativen Erkrankungen, bei Leukämikern nach Bestrahlung, bei koronararteriellen Erkrankungen oder bei Lungenembolie.
Die Langzeit-Endothelschäden und Langzeit-Endothelzerstörungen sind solche, vorzugsweise bei Atherosklerose, bei stabiler und instabler Angina pectoris, bei Diabetes mellitus, bei Rheuma, bei Rheumatoide Arthritis, bei Multiple sclerose, bei Myasthenia Gravis, bei Psoriasis vulgaris, bei Graves disease, bei Morbus Basedow, bei Goodpasture-Syndrom, bei Idiopathische Thrombozytopenie Purpura (ITP), bei Aplastische Anämie, bei Inflammatorischer Bowel Erkrankung, bei Morbus Crohn, bei Colitis ulcerosa, bei Dilatative Cardiomyopathie (DCM), bei Autoimmuner Thyroiditis, bei Hashimoto's Thyroiditis, bei Hormon-Ersatz-Therapien (HRT) als auch bei Osteoarthritis.

Bei den Verbindungen, welche Eigenschaft besitzen, CRP zumindest temporär zu binden, handelt es sich um Verbindungen ausgewählt aus der Gruppe umfassend oder bestehend aus Lipiden, Lysophospholipiden, Lysophosphatidylcholinen, Peptiden, Peptiden mit geladenen Aminosäuren, Peptiden enthaltend die Sequenz ArgProArg, Polypeptiden, Antikörpern, monoklonalen Antikörpern, Antikörperfragmenten, manipulierten Antikörpern, Phosphocholinen, Derivaten von Phosphocholin, Phosphatidylserine, Serinkephaline, DNA, DNA-Derivaten und Aptameren.

Bevorzugt sind Phosphocholine und Phosphoethanolamine der allgemeinen Formel I 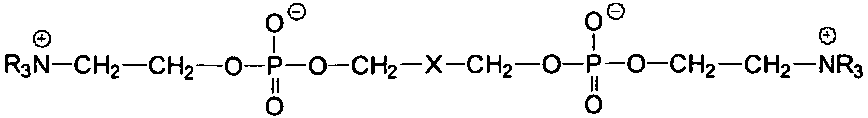 worin
X für eine chemische Einfachbindung oder einen Alkylrest mit 1 bis 20 Kohlenstoffatomen, bevorzugt 1 bis 12 Kohlenstoffatomen oder einen Arylrest mit 6 bis 18 Kohlenstoffatomen oder einen Cycloalkylrest mit 3 bis 7 Kohlenstoffatomen steht oder einen Alkylcycloalkylrest mit 3 bis 20 Kohlenstoffatomen, bevorzugt 3 bis 12 Kohlenstoffatomen oder einen Arylalkylrest mit 7 bis 20 Kohlenstoffatomen oder einen Aryldialkylrest mit 8 bis 20 Kohlenstoffatomen und
die Reste R unabhängig voneinander für Wasserstoff oder lineare oder verzweigte sowie gesättigte oder ungesättigte Alkylreste mit 1 bis 10 Kohlenstoffatomen stehen sowie enantiomere Formen, Racemate, Enantiomerengemische, Diastereomerengemische, Salze, Hydrate, Solvate und Regioisomere der vorgenannten Verbindungen.
Die Alkyl-, Aryl-, Cycloalkyl-, Alkylcycloalkyl-, Arylalkyl- und Aryldialkylreste können ferner mit einer oder mehreren funktionellen Gruppen wie z.B. -OH, -OCH₃, -OC₂H₅, -SH, -NO₂, -F, -Cl, -Br, -I, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₂F, -CHF₂, -CF₃, -CH₂Cl, -CH₂Br, -CH₂I, -Ph, -CH₂-Ph, -CH₃, -C₂H₅, -C₃H₇, -CH=CH₂, -CH₂-CH=CH₂, -C≡CH substituiert sein und/oder ein oder mehrere Heteroatome wie z.B. O, S, N, P enthalten.

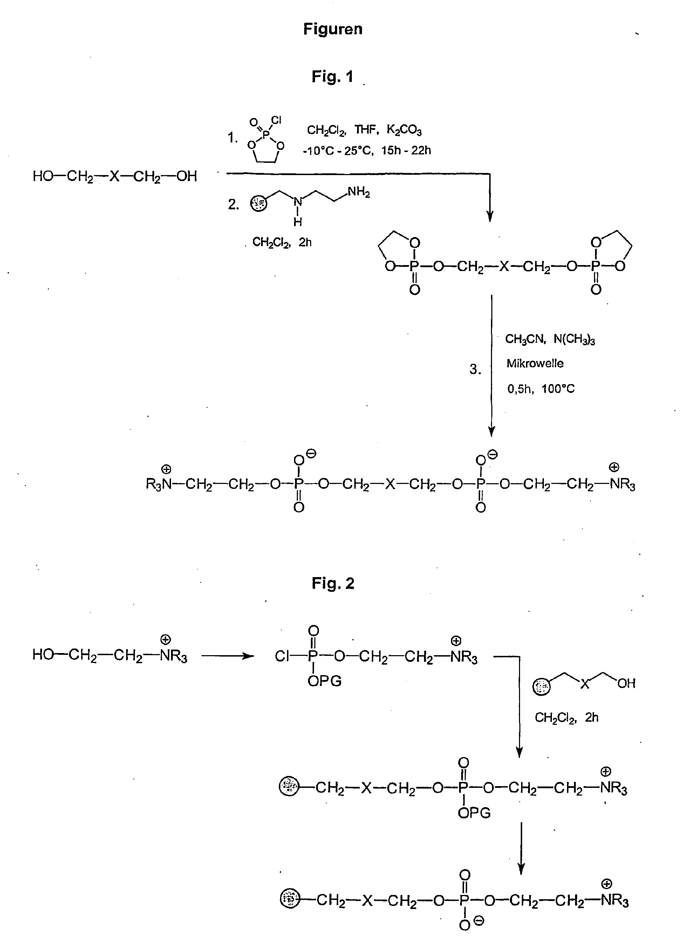

Figur 1 zeigt eine mögliche Synthese für derartige Phosphocholine.
Explizite Beispiele sind folgende Verbindungen: 1,5-Bis(phosphocholin)pentan, 1,6-Bis(phosphocholin)hexan, 1,7-Bis(phosphocholin)hepan, 1,4-Bis(phosphocholin)dimethylcyclohexan. Neben den Bisphosphocholinen können natürlich auch Tris-, Tetra- und Pentaphosphocholine eingesetzt werden.

Des weiteren sind insbesondere Phosphocholine bzw. Phosphoethanolamine und Phosphoserine der folgenden allgemeinen Formel (IIA) und (IIB) bevorzugt, welche kovalent an einen festen Träger gebunden werden:

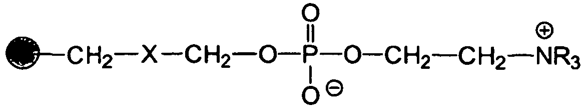

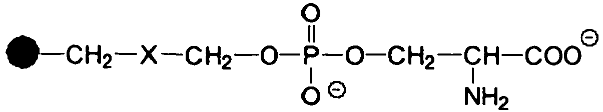

Figur 2 zeigt einen möglichen Syntheseweg für derartige Phosphocholine. X stellt einen beliebigen Linker dar und bevorzugt Linker der obigen Definition für X. Zusätzlich zu den obigen Definitionen für X kann dieser Linker noch folgende Gruppe enthalten: -O-, -S-, -NH-, -CH=CH-, -C≡C-, -O-(CH₂)ₘ-, -NH-(CH₂)ₚ-, -CO-, -CO-O-, -NH-CO-, -CO-NH-, -O-CO-, -O-CO-O-.

Des weiteren sind Glycerophosphocholine bzw. Glycerophosphoethanolamine als auch Glycerophosphoserine der allgemeinen Formeln (IIIA), (IIIB), (IIIC), (IIID), (IIIE), (IIIF), (IIIG), (IIIH) bevorzugt, welche an einen festen Träger kovalent gebunden sind:

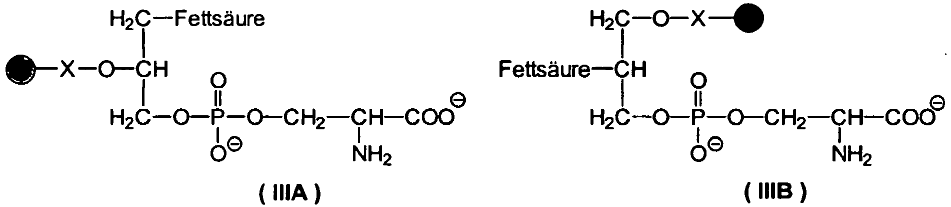

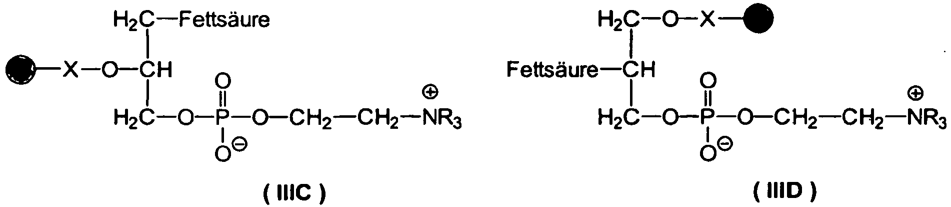

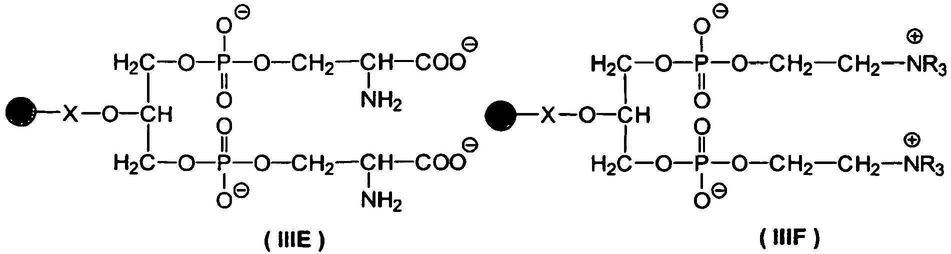

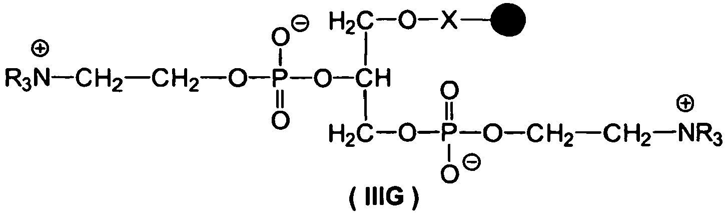

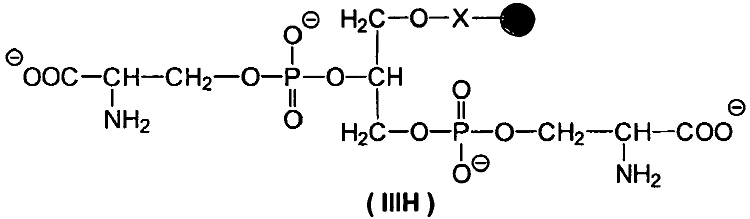

Als Fettsäuren können die üblichen gesättigten, monoolefinischen, polyolefinischen, monoacetylenisch, ungesättigten linearen und/oder verzeigten Fettsäuren mit 8 bis 28 Kohlenstoffatomen dienen. Bevorzugte Fettsäurereste sind Palmitoyl, Arachidonoyl, Oxovaleroyl, Glutaroyl, Epoxyisoprostan, Stearoyl. Die Fettsäurereste sind über eine Estergruppe (COO) an das Glycerin gebunden. -CH₂-Fettsäure bedeutet somit -CH₂-O-CO-R', wobei R' die Kohlenstoffkette der Fettsäure darstellt.

Die genannten Bisphosphocholinverbindungen, Bisphosphoethanolaminverbindungen, Phosphocholine, Phosphoethanolamine, Glycerophosphocholine, Phosphoserine, Glycerophosphoethanolamine als auch Glycerophospho serine können ionisch an einen festen Träger immobilisiert werden oder mittels vernetzender Verbindungen wie Polymeren auf der Oberfläche von festen Trägern immobilisiert werden. Als feste Träger, welche auch als die hierin erwähnten pharmazeutischen Mittel bezeichnet werden, können die hierin genannten Partikel oder Fasern oder Granulate aus Keramiken, Glas, Polymeren, Aluminiumoxid, Siliuiumoxid, Kieselgel, Sepharose usw. genannt werden. Die festen Träger werden im den Figuren als Kreis mit Strichen dargestellt.

Diese vorgenannten Verbindungen werden ferner vorzugsweise kovalent direkt oder über einen Linker oder ionisch oder adsoptiv an einen Träger gebunden oder in eine Polymerschicht auf einem Träger eingelagert oder an eine solche Polymerschicht gebunden. Beispiele für solche Träger sind matrixbildende Substanzen und Matrixsubstratmaterialien wie beispielsweise Eupergit, Polysulfon, Polyvinylpyrollidon, formatiertes Silicon, Sepharose, Acrylbeads, Agarose, Zellulosematrizen, Keramikmatrizen, Glassbeads und/oder Festphasen-Kieselerde oder Mischungen und/oder Derivate dieser Stoffe. Als Matrix wie hierin verwendet wird eine mit Verbindungen, welche die Eigenschaft haben, CRP zumindest temporär zu binden, beladene Matrixsubstratmaterialien verstanden.

Grundsätzlich sind für die Herstellung der Matrix sämtliche inerten Chromatographie- oder Säulenmaterialien geeignet, welche insbesondere nicht mit Blut oder Blutplasma reagierten oder Blut bzw. Blutplasma derart verändern oder kontaminieren, dass es nach Kontakt mit der Matrix einem Patienten nicht mehr injiziert werden kann. Als Verbindungen kommen grundsätzlich alle in Frage, welche sich an Matrixsubstratmaterialien immobilisieren lassen und in der Lage sind, CRP zumindest zeitweise reversibel oder auch dauerhaft irreversibel zu binden.

Ferner wurde gefunden, dass es nicht immer erforderlich ist, CRP vollständig, d.h. mehr als 90% aus der biologischen Flüssigkeit zu entfernen. Teilweise wird der therapeutische oder prophylaktische Erfolgt bereits erhalten, wenn der CRP-Spiegel wieder auf einen normalen Wert gesenkt wird.

Somit beschreibt diese Offenbarung auch ein Verfahren zur Entfernung oder Reduzierung von CRP in biologischen Flüssigkeiten dadurch, dass die biologische Flüssigkeit mit der Matrix in Kontakt gebracht wird und die Verbindungen, welche die Eigenschaft haben, CRP zumindest temporär zu binden, CRP zumindest temporär binden. Nach der Trennung der biologischen Flüssigkeit wie beispielsweise Blut oder Blutplasma von der Matrix wird biologische Flüssigkeit mit einem reduzierten oder deutlich verringerten Gehalt an CRP erhalten, welches dann einem Patienten, der dafür Bedürfnis hat, wieder mittels Infusion verabreicht werden kann.

Die vorliegende Offenbarung_umfasst weiterhin eine Methode und ein System zur Entfernung von C-reaktivem Protein aus besagten biologischen Flüssigkeiten, welche das Pumpen von Patientenblut durch ein Gerät umfaßt, welches adsorbierendes Matrixmaterial enthält, das spezifisch CRP bindet und CRP aus dem Blut entfernt, wodurch behandeltes Plasma produziert wird, und die Rückgabe des behandelten Blutes in den selben Patienten und/oder das Pumpen von Blut eines Patienten durch einen Zellseparator, welcher Zellen in Blutzellen und Plasma separiert, die Plasmakomponenten durch einen Apparat wie z. B. eine Säule passiert, welche adsorbierendes Matrixmaterial wie z. B. Phosphocholin zur Entfernung von C-reaktivem Protein enthält, und Zusammenführung des behandelten Plasma und der Zellen vor der Rückgabe des selben in den Patienten.

Der weitere Anwendungsbereich der vorliegenden Erfindung wird aus der nachstehend aufgeführten detaillierten Beschreibung offenbar. Es sollte aber verstanden werden, daß die detaillierte Beschreibung, obwohl bevorzugte Ausführungsbeispiele der Erfindung aufzeigend, nur als erläuternde Illustration zu verstehen ist, da verschiedene Änderungen und Modifikationen im Geiste und im Rahmen der Erfindung offensichtlich für jene sein werden, die Fachleute für diese detaillierte Beschreibung sind.

### Detaillierte Beschreibung der Erfindung

Das Ziel der vorliegenden Erfindung ist, Werkzeuge, Moleküle und Methoden zur Verringerung der CRP Mengen in Menschen zur Verfügung zu stellen. Dieses Ziel wird erreicht durch die Verwendung eines Gemisches, welches mindestens eine strukturelle Entität enthält, welche CRP bindet oder ein CRP-Antagonist ist, dieses Gemisch depletiert CRP aus einer Lösung oder blockt mindestens eine oder mehr CRP-Funktionen auf Zelloberflächen oder in einer Lösung, zur Herstellung einer Matrix zur extrakorporalen Behandlung von biologischen Flüssigkeiten für die Behandlung oder Prävention von Erkrankungen, welche selektiert sind aus der Gruppe bestehend aus Herzinfarkt und Schlaganfall.

Die vorliegenden Erfindung basiert auf der Erkenntnis das Moleküle, welche CRP binden, z.B., Lipide, bevorzugt Lysophospholipide, vorzugsweise Lysophosphatidylcholin, Peptide, bevorzugt solche, die geladene Aminosäuren enthalten, besonders mit einem ArgProArg-Rückgrat oder mit der Sequenz Gly-Pro-Arg-Pro-Lys, Polypeptide, bevorzugt Antikörper, besonders monoklonale Antikörper, Antikörperfragmente oder manipulierte Antikörper, ein rekombinanter Antikörper (wie z.B. Einzelkettenantikörper - scAb oder scFv; bispezifische Antikörper, Diabody), Phosphocholin (PC) oder PC-Derivate, nützlich sind für die Verringerung der Menge an CRP.

So, wie er hier verwendet wird, bedeutet der Terminus "effektive Menge" eine Menge eines Gemisches oder eines Moleküls, welche CRP binden, welche in der Lage ist, die Menge an CRP zu verringern und/oder Zustände oder schädliche Wirkungen zu inhibieren, die hervorgerufen werden durch ein Übermaß an CRP.

Der Ausdruck 'Östrogen-Mangel' bezieht sich auf einen Zustand, entweder natürlich vorkommend oder klinisch bewirkt, in dem eine Frau nicht genug Östrogenhormone produzieren kann, um Östrogen-abhängige Funktionen, wie Mensis, Erhaltung der Knochenmasse, neuronale Funktionen, kardiovaskuläres Befinden, etc., aufrecht zu erhalten. Solche Östrogen-Mangel-Situationen entstehen durch, sind aber nicht beschränkt darauf, Menopause und chirurgische oder chemische Eierstockentfernung, einschließlich seiner funktionalen Entsprechung wie Behandlung mit GnRH Agonisten oder Antagonisten, ICI 182780 und ähnlichem.

Der Ausdruck 'verhindern' im Zusammenhang mit der Verhinderung von Zuständen oder schädlichen Wirkungen, die durch ein Übermaß an CRP verursacht werden, beinhaltet seine allgemein akzeptierte Bedeutung wie z. B. blockieren, vermeiden, unterdrücken, lindern, verbessern, verlangsamen, beenden oder umkehren des Voranschreitens oder der Schwere einer Zunahme an CRP und der pathologischen Folgen, z. B. Symptome, die aus diesem Ereignis hervorgehen.

Der Ausdruck 'pharmazeutisch', wenn er hier als Adjektiv benutzt wird, bedeutet, im Wesentlichen nicht-toxisch und im Wesentlichen nicht schädlich für den Empfänger.

Bei 'pharmazeutischer Formulierung' oder 'Medikament' oder 'pharmazeutische Zusammensetzung' ist weiterhin gemeint, daß der Träger, das Lösungsmittel, der Arzneistoffträger und Salze mit den aktiven Inhaltsstoffen der Formulierung verträglich sein müssen (eine Zusammensetzung aus mindestens einem Molekül, welches CRP bindet).

Der Begriff 'Lösung' repräsentiert eine Zusammensetzung, die aus einem oder mehreren Molekülen des zu lösenden Stoffes besteht, mit einem oder mehreren Molekülen eines pharmazeutischen Lösungsmittel, wie z. B. Wasser, Puffer, physiologische Salz Lösung und ähnliches.

In einem Ausführungsbeispiel ist die Substanz-, welche die Eigenschaft hat, humanes CRP zumindest temporär zu binden ein Polypeptid, welches eine Bindungsstelle für CRP enthält, bevorzugt ein Antikörper, der eine Antigen-Bindungsstelle für CRP enthält. Die Substanz, welche die Eigenschaft hat, humanes CRP zumindest temporär zu binden, ist im besonderen ein poly- oder monoklonaler Antikörper, der eine Antigen-Bindungsstelle für CRP enthält.

Der monoklonale Antikörper enthält insbesondere eine Antigen-Bindungsstelle für CRP und wird nach Immunisierung von Wirbeltieren erhalten, bevorzugt Säugetieren, wie Mäuse, Ratten, Meerschweinchen, Hamster, Affen, Schweine, Ziegen, Hühner, Kühe, Pferde und Kaninchen. Der poly- oder monoklonale Antikörper, der eine Antigen-Bindungsstelle für CRP enthält, ist bevorzugt humanisiert entsprechend Technologien, die Fachleuten bekannt sind. Die Substanz kann auch erzeugt werden durch die Immunisierung von humanisierten Mäusen und/oder immundefizienten Mäusen (wie z. B. SCID oder Nackt-Mäuse), die mit vitalen Immunzellen (z. B. humanen Ursprungs; wie z. B. SCID-hu Mäuse) repopuliert wurden.

In einem weiteren Ausführungsbeispiel ist der Antikörper ein rekombinanter Antikörper (wie z. B. Einzelketten-Antikörper - scAb oder scFv; bispezifische Antikörper, Diabodys, usw.), fähig dazu, an CRP zu binden, besonders dadurch, dass er eine Antigen-Bindungsstelle eines Antikörpers enthält, welcher mit CRP kreuzreagiert. Das Antikörper Molekül ist ein humanisierter oder humaner Antikörper.

Gegenstand der Beschreibung ist auch eine Methode zur Produktion von rekombinanten Molekülen, die, fähig sind, an das CRP Antigen zu binden, welche den Schritt der Produzentenzellenkultur und der Isolierung des Bindemoleküls aus dem Kulturmedium oder der Produzentenzelle enthält.

Ein anderer Gegenstand betrifft eine pharmazeutische Zusammensetzung zur Reduktion der CRP Konzentration und/oder der unbesetzten CRP Konzentration, die einen therapeutisch und/oder für Apherese und/oder Dialyse wirksamen Anteil an den Bindemolekülen einen pharmazeutisch akzeptablen Träger enthält. Zusätzlich zu diesen Substanzen kann das Medikament anti-inflammatorische Substanzen enthalten die ausgesucht sind aus der Gruppe bestehend aus Molekülen, welche Phospholipase A2 (PLA2), sekretorische PLA2 (sPLA2) Typ II, sPLA2 Typ IIA, Interleukin-6, Interleukin-1, Interleukin-15 und/oder Tumor Nekrose Faktor alpha (TNFα) binden, und/oder Antagonisten, CRP bindende Moleküle, Anti-IL-1ß-Moleküle, PLA2 Antagonisten, PLA2 bindende Moleküle, Komplementblocker, mindestens aber ein weiteres Bindemolekül für die Behandlung von inflammatorischen Reaktionen in kardiovaskulären Risikopatienten wie Patienten mit akuten koronaren Syndromen, Herzinfarkten, mit Niereninsuffizienz, oder Dialysepatienten oder Kombinationen davon.

Ein weiterer Gegenstand der Offenbarung ist eine Methode zur Reduktion von entzündlichen Immun- und/oder pathophysiologischen Reaktionen durch Reduktion der CRP-Konzentration, eine Methode zur Reduktion von Endothel-Verletzungen und/oder -Zerstörungen durch Reduktion der CRP-Konzentration, eine Methode zur Akutbehandlung im Fall von akuter Endothel-Verletzungen und/oder - Zerstörungen, besonders bei Schlaganfall, Herzinfarkt, Verhinderung des plötzlichen Herztodes, bei Verbrennungen, bei schweren Operationen oder anderen Verletzungen mit großen Wundbereichen, bei diabetischem Schock, bei akutem Leberversagen, bei Pankreatitis, bei neurodegenerativen Krankheiten, bei leukämischen Personen nach Bestrahlung, eine Methode für Dauerbehandlungen im Fall von langwieriger Endothel-Verletzung und/oder -Zerstörung, besonders für Patienten mit mittlerem (medium) CRP Gehalt, mit Arteriosklerose, mit unstabiler Angina, mit Diabetes Typ I oder II, mit Übergewicht und/oder Fettleibigkeit, für Alkoholiker, während Hormon-Ersatz-Therapie (HRT), für alte Leute, für Raucher, eine Methode zur Verhinderung von Allo-Transplantat oder Xeno-Transplantat Abstoßung, eine Methode zur Induzierung von Allo-Transplantat oder Xeno-Transplantat-Toleranz oder Inhibierung der T-Zell-Aktivierung und eine Methode zur Verhinderung oder Behandlung von Autoimmunkrankheiten, die Methoden beinhalten die Behandlung durch Apherese oder Dialyse mit einer therapeutisch effektiven Dosis einer pharmazeutischen Zusammensetzung/Matrix an einen Patienten, der eine solche Behandlung benötigt.

Die Substanz/Matrix kann mit anderen Molekülen kombiniert werden, bevorzugt Therapeutika für die entsprechende Krankheit oder andere anti-inflammatorische Substanzen wie z.B. Moleküle, welche Serumcholesterin, HDL, LDL binden oder andere anti-inflammatorische Moleküle, wie z.B. Moleküle, die IL-6 und/oder den IL-6-Rezeptor, Anti-IL-1-Moleküle binden, IL-15-Antagonisten, PLA2-Antagonisten, PLA2 bindende Moleküle, Komplementblocker, C-reaktives Protein (CRP) Antagonisten, CRP bindende Moleküle, Anti-IL-1ß-Rezeptor-Moleküle, PLA2 Antagonisten, mindestens aber ein weiteres Bindemolekül für die Behandlung von inflammatorischen Reaktionen in kardiovaskulären Risikopatienten wie Patienten mit akuten koronaren Syndromen, Herzinfarkten, mit Niereninsuffizienz, oder Dialysepatienten oder Kombinationen davon.

Die Methoden, vorliegende sind nützlich sowohl für die Behandlung als auch die Verhinderung von schädlichen Folgen die verbunden sind mit erhöhten CRP-Werten. Da CRP-Serumkonzentrationen in Beziehung stehen zu Konzentration und Produktion von Zytokinen, die besonders während entzündlicher Prozesse produziert werden, sind die Methoden nützlich für die Behandlung oder Verhinderung entzündlicher Ereignisse und ihrer Folgen. Solche entzündlichen Ereignisse beinhalten, sind aber nicht beschränkt auf: Niereninsuffizienz, Dialyse, Arthritis (Osteoarthritis), arterielle und venöse chronische Entzündung, Autoimmunkrankheiten, z.B. SLE, Multiple Sklerose, Myasthenia Gravis, Graves Erkrankung (Morbus Basedow), Psoriasis vulgaris, Dilatative Cardiomyopathie, Diabetes Mellitus, Morbus Bechterew, Entzündliche-Gallen-Krankheit, Ulcerative Colitis, Morbus Crohn, Idiopathische Thrombocytopenia Purpura (ITP), aplastische Anämie, Idiopathische Dilatative Cardiomyopathie (IDM), autoimmune Thyroiditis, Goodpastures' Krankheit und ähnliche.

Die Methoden sind nützlich für die Behandlung oder Verhinderung pathologischer Folgen von arteriosklerotischen oder thrombotischen Krankheiten. Solche Pathologien beinhalten, sind aber nicht beschränkt auf, Schlaganfall, Kreislaufversagen, Ischämische Ereignisse, Herzinfarkt, Lungenembolie, stabile und instabile Angina, Herzkranzgefäß-Erkrankungen, plötzlicher Tod-Syndrom und ähnliches.

Weiterhin können die Substanzen/Matrizen mit mindestens einem Molekül, das CRP bindet entweder in einer Behandlung oder in vorbeugender Anwendung eingesetzt werden.

Ein Möglichkeit ist, dass der Zustand, der durch abnormal hohe CRP-Spiegel verursacht wurde, eine kardiovaskuläre Erkrankung ist, besonders Arteriosklerose Atherosklerose, Stenose, Restenose und Thrombose oder andere Akutbehandlungen im Fall von akuten Endothel-Verletzungen und/oder - Zerstörungen, wie Schlaganfall, Herzinfarkt, plötzlicher Herztod, Verbrennungen, schwere Operationen oder andere Verletzungen mit großem Wundbereichen, diabetischer Schock, akutes Leberversagen, Pankreatitis, neurodegenerative Krankheiten, leukämische Personen nach Bestrahlung oder langwierige Endothel-Verletzung und/oder Zerstörung, wie Arteriosklerose, Diabetes Typ I oder II, Übergewicht und/oder Fettleibigkeit, Alkoholismus, Hormon-Ersatz-Therapie (HRT), alte Leute, Raucher.

Pharmazeutische Zusammensetzungen/Matrizen können durch fachlich bekannte Verfahren hergestellt werden, so dass z. B. eine Substanz/Matrix hergestellt werden kann, mit mindestens einem Molekül, das CRP bindet, mit gewöhnlichen Zusätzen, Lösungsmittel oder Trägern und die zu Kugeln, Fäden und ähnlichem geformt wird.

Beispiele der besagten Adsorbermatrix sind Matrixsubstratmaterialien ausgewählt aus der Gruppe bestehend aus Eupergit, Polysulfon, Polyvinylpyrollidon, formatiertes Silicon, Sepharose, Acrylbeads, Agarose, Zellulosematrizen, Keramikmatrizen, Glassbeads und/oder Festphasen Kieselerde oder Derivate davon.

### Prozess zur Entfernung von CRP aus biologischen Flüssigkeiten

Die vodiegende Offenbarung befasst sich mit einer Methode zur Entfernung von C-reaktivem Protein aus biologischen Flüssigkeiten, um deren inflammatorische Parameter zu verbessern, und spezifisch mit einer Methode zur Entfernung von C-reaktivem Protein durch eine extrakorporale Perfusion von Patienten Blutplasma durch eine Phosphocholin-Matrix enthaltende Adsorptionsapparatur, um dadurch die kardiovaskulären Parameter des Patienten zu verbessern. Die CRP-bindende Matrix kann enthalten Lipide, insbesondere Lysophospholipide, bevorzugt Lysophosphatidylcholin, Peptide, insbesondere solche, die geladene Aminosäuren besonders mit einem ArgProArg-Rückgrat enthalten, Polypeptide, insbesondere Antikörper besonders monoklonale Antikörper, Antikörperfragmente oder manipulierte Antikörper und/oder PC und/oder PC-Derivate, DNA oder DNA-Derivate (z.B. Aptamere).

Eine weitere Möglichkeit ist die Entfernung von C-reaktivem Protein aus dem Blut von Patienten durch extrakorporale Perfusion von deren Blutplasma durch eine Phosphocholin-Matrix enthaltende Adsorptionsapparatur. Die CRP-bindende Matrix kann enthalten Lipide, insbesondere Lysophospholipide, bevorzugt Lysophosphatidylcholin, Peptide, insbesondere solche, die geladene Aminosäuren besonders mit einem ArgProArg-Rückgrat enthalten, Polypeptide, insbesondere Antikörper besonders monoklonale Antikörper, Antikörperfragmente oder manipulierte Antikörper und/oder PC und/oder PC-Derivate, DNA oder DNA-Derivate (z.B. Aptamere).

Eine weitere Möglichkeit ist es, ein System für das Pumpen von Patientenblut durch einen Zellseparator bereit zu stellen, welcher Blut zu Zell- und Plasmakomponenten separiert, welches die Schritte beinhaltet, die Plasmakomponenten durch einen Apparat zu passieren, welcher adsorbierendes Matrixmaterial wie Phosphocholin zur Entfernung von C-reaktivem Protein enthält, wodurch behandeltes Plasma produziert wird. Die Zusammenführung des behandelten Plasma und der Zellkomponenten, um behandeltes Blut zu produzieren und die Rückgabe des behandelten Blutes in den Patienten ist auch enthalten.

Eine andere Möglichkeit ist die Entfernung von löslichem C-reaktivem Protein aus dem Blut von Patienten durch extrakorporale Perfusion ihres gesamten Blutes durch eine Phosphocholin-Matrix beherbergende Adsorptionsapparatur. Die CRP-bindende Matrix kann enthalten Lipide, insbesondere Lysophospholipide, bevorzugt Lysophosphatidylcholin, Peptide, insbesondere solche, die geladene Aminosäuren besonders mit einem ArgProArg-Rückgrat enthalten, Polypeptide, insbesondere Antikörper besonders monoklonale Antikörper, Antikörperfragmente oder manipulierte Antikörper und/oder PC und/oder PC-Derivate, DNA oder DNA-Derivate (z.B. Aptamere).

Ein weiterer Aspekt der Offenbarung ist ein System, beihaltend eine Blutpumpe für das Pumpen von Patientenblut zu einer Säule oder Apparatur, welche mit der besagten Pumpe verbunden ist, welche adsorbierendes Matrixmaterial enthält, beinhaltend Lipide, insbesondere Lysophospholipide, bevorzugt Lysophosphatidylcholin, Peptide, insbesondere solche, die geladene Aminosäuren besonders mit einem ArgProArg-Rückgrat enthalten, Polypeptide, insbesondere Antikörper besonders monoklonale Antikörper, Antikörperfragmente oder manipulierte Antikörper und/oder PC und/oder PC-Derivate, DNA oder DNA-Derivate (z.B. Aptamere), zur Entfernung von C-reaktivem Protein, wodurch behandeltes Blut produziert wird, welches dann in den Patienten zurückgegeben wird.

Um die Methoden auszuführen wird ein extrakorporales Gerät oder Säule zur Verfügung gestellt, beinhaltend eine Matrix mit CRPadsorbierendem Material für die extrakorporale Behandlung einer biologischen Flüssigkeit, wie z. B. humanes Blut und Blutplasma, Peritoneal-Flüssigkeit und lymphatische Flüssigkeit, um CRP daraus zu entfernen. Die Behandlung kann so durchgeführt werden, daß kontinuierlich das Patientenblut entfernt wird, die Blutzellen daraus separiert werden, das separierte Blut in der CRP-adsorbierenden Säule oder Gerät behandelt wird, um das CRP zu entfernen, danach das behandelte Plasma und die Blutzellen gemischt und dem Patienten direkt zurückgegeben werden. Alternativ können, nachdem das Blut von den separierten Blutzellen entfernt wurde, die Blutzellen direkt in den Patienten reinfundiert werden. Das separierte Plasma kann gesammelt, in der CRP-adsorbierenden Säule behandelt, und dann so schnell wie möglich in den Patienten zurückgegeben werden.

Das CRP-adsorbierende Material, welches sich in der Säule befindet, beinhaltet Lipide, insbesondere Lysophospholipide, bevorzugt Lysophosphatidylcholin, Peptide, insbesondere solche, die geladene Aminosäuren besonders mit einem ArgProArg-Rückgrat enthalten, Polypeptide, insbesondere Antikörper besonders monoklonale Antikörper, Antikörperfragmente oder manipulierte Antikörper und/oder PC und/oder PC-Derivate, DNA oder DNA-Derivate (z.B. Aptamere) gebunden an eine Matrix, welche die Aktivität des PC und/oder PC-Derivates und die Bindungskapazität der Säule oder des Gerätes maximiert, während sie das Herauslösen von PC und/oder PC-Derivat, sowie anderen Substanzen aus der Säule während des Gebrauches minimiert.

Generell wird eine effektive Menge von CRP-bindenden Substanzen verwendet, um eine Säule zu präparieren. Zum Beispiel werden ein paar Milligramm PC oder andere CRP-bindende Substanzen pro Gramm Matrixmaterial verwendet. Ein Beispiel eines nicht limitierenden Bereiches ist von ungefähr 0.06 bis 1.6 mg der Substanz pro Gramm Säulen Matrixmaterial. In einem bevorzugten Ausführungsbeispiel werden PC oder PC Derivative mit Aminogruppen von formatierter Siliconmatrix kreuzvernetzt, um in der Lage zu sein CRP zu entfernen, und inflammatorische Parameter zu verbessern.

Die PC und PC Derivative, welche nützlich für die Verwendungen der vorliegenden Erfindung sind, umfassen alle PC-Derivative, welche genügend gut CRP binden. Beispiele von PC-Derivativen beinhalten PC-Ester, wie p-Nitrophenyl-6-(O-Phosphocholin)hydroxyhexanoate und p-Aminophenylphosphocholin.

Die Matrix, welche sich in dem extrakorporalen Gerät oder Säule befindet, kann aus jeglichem Material welches nützlich ist, CRP-bindende Substanzen zu tragen, geformt werden, wie z. B. Silicon, Agarose, Sepharose, Acrylkugeln, andere geeignete Polymere Substanzen und Matrizes, und Festphasen-Kieselerde. Die Festphasen-Kieselerde-Matrix kann nahezu jede Form von partikulärer Kieselerde umfassen einschließlich amorpher Kieselerden, wie kolloidale Kieselerde, Kieselgele, präzipitierte Kieselerden, und geräucherte oder pyrogene Kieselerden; microkrystalline Kieselerden wie Kieselgur; und krystalline Kieselerden wie Quartz.

Die Kieselerde sollte eine Partikelgröße im Bereich von ungefähr 45 bis 120 Öffnungen pro inch² haben, normalerweise in dem Bereich von ungefähr 45 bis 60 Öffnungen pro inch². Andere Materialien, welche nützlich für die Bildung einer Matrix sind, wie in U.S. Patent 4,681,870 offenbart, können auch in dem Gerät oder der Säule verwendet werden, . U.S. Patent 4,681,870 ist hiermit durch Referenz aufgenommen.

Die CRP-bindenden Substanzen sind an das Gerät oder die Säulenmatrix in einer geeigneten Art gebunden, so daß die Fähigkeit der CRP-bindenden Substanzen, CRP aus biologischen Flüssigkeiten zu entfernen, erhalten bleibt. Zum Beispiel können die CRP-bindenden Substanzen mit Aminogruppen einer formatierter Siliconmatrix kreuzvernetzt sein. Andere Methoden, um PC oder PC Derivate an das Matrixmaterial zu binden, wie die zutreffenden Methoden offenbart in U.S. Patent 4,681,870, können angewendet werden.

Die Methode kann durchgeführt werden durch die Verwendung eines geeigneten extrakorporalen Gerätes oder Säule, die das oben beschriebene CRP-adsorbierende Matrixmaterial enthält. Die Säule kann eine herausnehmbare Patrone enthalten, welche das adsorbierende Matrixmaterial enthält. Ein Beispiel für eine solche Patrone ist in U.S. Patent 4,681,870 beschrieben. Eine Säule oder ein Gerät ist mit einem Zellseparator verbunden. Die Säule oder das Gerät kann sterilisiert werden, zum Beispiel mit einem sterilisierenden Gas wie Ethylenoxid, und kann dann entweder gleich verwendet oder verschlossen und gelagert werden.
Vor der Verwendung kann die Säule oder das Gerät mit normaler Salzlösung gespült werden, gefolgt durch Spülen mit normaler Salzlösung, die jegliche anderen passenden Vorbereitungsinhaltsstoffe enthalten kann. Jedoch sollten keine Kalziumionen-chelatierenden Agenzien bei PC oder LysoPC eingebracht werden.

Die Säule oder das Gerät wird dann mit dem Zellseparator verbunden, um von diesem separiertes Plasma zu erhalten. Der Zellseparator kann ein Durchlauf-Zellseparator sein, oder kann eine semi-permeable Membran umfassen, welche die Passage des Plasmas und von Blutprotein erlaubt, aber die Passage von zellulären Elementen des Blutes verhindert. Im Falle einer semi-permeablen Membran wird eine Blutpumpe verwendet, um das Blut durch die Membran zu passieren. Geeignete Blutpumpen enthalten eine Röhre und eine peristalische Pumpe, in welcher das Blut von der pumpenden Maschinerie isoliert wird, um Kontaminationen zu verhindern. Das Blut passiert einen Zellseparator mit einer Rate, welche im Bereich von etwa 10 bis 500 ml/min liegen kann, typischerweise bis ein totales gewünschtes Volumen Blut durchgelaufen ist. Die Blutzellen werden mit dem Plasma gemischt, welches durch die Behandlungsäule oder das -gerät gelaufen ist, und das wiedervereinte Blut wird dem Patienten zurück gegeben. Ein Mikrofilter kann am Auslaß der Behandlungssäule oder -gerät verwendet werden, um die Passage von makroskopischen Partikeln zu verhindern, welche von der Säule oder dem Gerät verloren gehen.

## Patentansprüche

1. Verwendung einer Matrix enthaltend Verbindungen, welche die Eigenschaft haben, humanes CRP zumindest temporär zu binden, zur Herstellung eines pharmazeutischen Mittels zur extrakorporalen Entfernung von humanem CRP aus biologischen Flüssigkeiten eines Patienten zur Prophylaxe und/oder Behandlung von Herzinfarkt und Schlaganfall, worin die Verbindungen, welche die Eigenschaft haben, humanes CRP zumindest temporär zu binden, ausgewählt werden aus der Gruppe umfassend Lipide, Lysophospholipide, Lysophosphatidylcholin, Peptide, Peptide mit geladenen Aminosäuren, Peptide enthaltend die Sequenz ArgProArg, Polypeptide, Antikörper, monoklonale Antikörper, Antikörperfragmente, manipulierte Antikörper, Phosphocholin, Derivate von Phosphocholin, DNA, DNA-Derivate und Aptamere, und
worin die Matrix des weiteren Matrixsubstratmaterialien umfasst, ausgewählt aus der Gruppe bestehend aus Eupergit, Polysulfon, Polyvinylpyrollidon, formatiertes Silicon, Sepharose, Acrylbeads, Agarose, Zellulosematrizen, Keramikmatrizen, Glassbeads und/oder Festphasen-Kieselerde oder Mischungen und/oder Derivate dieser Stoffe.

2. Verwendung von Verbindungen, welche die Eigenschaft haben, humanes CRP zumindest temporär zu binden, zur Herstellung einer pharmazeutischen Matrix zur extrakorporalen Entfernung von humanem CRP aus biologischen Flüssigkeiten eines Patienten zur Prophylaxe und/oder Behandlung von Herzinfarkt und Schlaganfall, worin die Verbindungen, welche die Eigenschaft haben, humanes CRP zumindest temporär zu binden, ausgewählt werden aus der Gruppe umfassend Lipide, Lysophospholipide, Lysophosphatidylcholin, Peptide, Peptide mit geladenen Aminosäuren, Peptide enthaltend die Sequenz ArgProArg, Polypeptide, Antikörper, monoklonale Antikörper, Antikörperfragmente, manipulierte Antikörper, Phosphocholin, Derivate von Phosphocholin, DNA, DNA-Derivate und Aptamere, und
worin die Matrix des weiteren Matrixsubstratmaterialien umfasst, ausgewählt aus der Gruppe bestehend aus Eupergit, Polysulfon, Polyvinylpyrollidon, formatiertes Silicon, Sepharose, Acrylbeads, Agarose, Zellulosematrizen, Keramikmatrizen, Glassbeads und/oder Festphasen-Kieselerde oder Mischungen und/oder Derivate dieser Stoffe.

3. Verwendung gemäß eines der vorherigen Ansprüche, worin die biologische Flüssigkeit menschliches Blut ist.

4. Verwendung gemäß eines der vorherigen Ansprüche, worin humanes CRP nicht vollständig aus der biologischen Flüssigkeit entfernt wird, sondern nur auf ein normales Maß reduziert wird.

## Claims

1. Use of a matrix comprising compounds having the property of binding at least temporary to human CRP, for the production of a pharmaceutical agent for the extracorporeal elimination of human CRP from the biological fluids of a patient for prophylaxis and/or treatment of heart attack or stroke, wherein the compounds, which have the property to bind to human CRP at least temporary, are selected from the group comprising lipids, lysophospholipids, lysophosphatidylcholines, peptides, peptides with charged amino acids, peptides containing the sequence Arg-Pro-Arg, polypeptides, antibodies, monoclonal antibodies, antibody fragments, engineered antibodies, phosphocoline, phosphocholine derivatives, DNA, DNA-derivatives and aptamers, and
wherein the matrix comprises further matrix substrate materials, which are selected from the group consisting of eupergit, polysulfone, polyvinylpyrrolidone, formatted silicon, sepharose, acryl beads, agarose, cellulose matrices, ceramic matrices, glass beads, and/or solid phase-silica or mixtures and/or derivatives of these materials.

2. Use of compounds, which have the property to bind at least temporary to human CRP, for the production of a pharmaceutical matrix for the extracorporeal elimination of human CRP from the biological fluids of a patient for prophylaxis and/or treatment of heart attack and stroke, wherein the compounds, which have the property to bind at least temporary to human CRP, are selected from the group comprising lipids, lysophospholipids, lysophosphatidylcholines, peptides, peptides with charged amino acids, peptides containing the sequence Arg-Pro-Arg, polypeptides, antibodies, monoclonal antibodies, antibody fragments, engineered antibodies, phosphocholine, phosphocholine derivatives, DNA, DNA-derivatives and aptamers, and
wherein the matrix contains further matrix substrate materials, which are selected from the group consisting of eupergit, polysulfone, polyvinylpyrrolidone, formatted silicon, sepharose, acryl beads, agarose, cellulose matrices, ceramic matrices, glass beads, and/or solid phase-silica or mixtures and/or derivatives of these materials.

3. Use according to one of the previous claims, wherein the biological fluid is human blood.

4. Use according to one of the previous claims, wherein the human CRP is not completely removed from the biological fluid, but only reduced to the normal value.

## Revendications

1. Utilisation d'une matrice contenant des composés ayant la propriété de s'attacher au moins temporairement à la protéine humaine C-réactive, pour la production d'un agent pharmaceutique pour l'élimination extracorporelle de la protéine humaine C-réactive des liquides biologiques d'un patient dans la prophylaxie et/ou le traitement de la crise cardiaque ou de l'accident vasculaire cérébral, où lesdits composés, qui ont la propriété de s'attacher au moins temporairement à la protéine humaine C-réactive, sont choisis dans le groupe contenant des lipides, des lysophospholipides, des lysophosphatidylcholines, des peptides, des peptides contenant des amino acides chargés, des peptides contenant la séquence Arg-Pro-Arg, des polypeptides, des anticorps, des anticorps monoclonaux, des fragments d'anticorps, des anticorps produits par génie génétique, de la phosphocholine, des dérivés de phosphocholine, de l'ADN, des dérivés d'ADN et des aptamères, et
où ladite matrice contient de plus, des matériaux substrats de matrice, qui sont choisis dans le groupe composé de l'eupergit, de la polysulfone, de la polyvinylpyrrolidone, du silicon formaté, de la sepharose, des sphères acryliques, des matrices de cellulose, des matrices de céramique, des sphères en verre et/ou de la silice en phase solide ou des mélanges et/ou des dérivés de ces matériaux.

2. Utilisation des composés, qui ont la propriété de s'attacher au moins temporairement à la protéine humaine C-réactive, pour la production d'une matrice pharmaceutique pour l'élimination extracorporelle de la protéine humaine C-réactive des liquides biologiques d'un patient dans la prophylaxie et/ou le traitement de la crise cardiaque ou de l'accident vasculaire cérébral, ou lesdits composés, qui ont la propriété de s'attacher au moins temporairement à la protéine humaine C-réactive, sont choisis dans le groupe contenant des lipides, des lysophospholipides, des lysophosphatidylcholines, des peptides, des peptides contenant des amino acides chargés, des peptides contenant la séquence Arg-Pro-Arg, des polypeptides, des anticorps, des anticorps monoclonaux, des fragments d'anticorps, des anticorps produits par génie génétique, de la phosphocholine, des dérivés de phosphocholine, de l'ADN, des dérivés d'ADN et des aptamères, et
où ladite matrice contient de plus, des matériaux substrats de matrice , qui sont choisis dans le groupe composé de l'eupergit, de la polysulfone, de la polyvinylpyrrolidone, du silicon formaté, de la sepharose, des sphères acryliques, des matrices de cellulose, des matrices de céramique, des sphères en verre et/ou de la silice en phase solide ou des mélanges et/ou des dérivés de ces matériaux.

3. Utilisation selon une des revendications précédentes, ou le liquide biologique est du sang humain.

4. Utilisation selon une des revendications précédentes, où ladite protéine humaine C-réactive n'est pas complètement éliminée du liquide biologique, mais seulement réduite à la valeur normale,
